# EUROPEAN PATENT APPLICATION

(11) **EP 4 545 131 A1**
(43) Date of publication of application: **30.04.2025**
(21) Application number: 22948102.3
(22) Date of filing: 21.10.2022
(51) Int. Cl.: A61M 27/00, A61M 1/00

(54) **DRAINAGE TUBE GUIDE DEVICE**

(30) Priority: 22.06.2022 KR 20220075924
(71) Applicant: JSR Medical CO., LTD., Daegu 42713 (KR); Kim, Jae Hwang, Daegu 42166 (KR)
(72) Inventor: KIM, Jae Hwang, Daegu 42166 (KR)
(74) Representative: Pfenning, Meinig & Partner mbB
(86) International application number: PCT/KR2022/016170
(87) International publication number: WO 2023/249168

(57) **Abstract**

There is disclosed a drainage tube guide device including an outer inserter inserted along the abdominal wall; and an inner inserter inserted in a hollow of the outer inserter while peeling tissues in front of the outer inserter, wherein the outer inserter may include a head part extending along one direction; and a guide tube extending downward from the head part.

## Description

### [BACKGROUND]

### [Technical Field]

The present disclosure relates to a drainage tube guide device.

### [Background of the Disclosure]

A drainage tube is inserted into the surgical site for the purpose of draining or observing body fluids, bleeding of wound secretions, accumulated secretions in the body and secretions after surgery or procedures.

At this time, such a drainage tube is fixed in the position desired by the surgeon. However, the drainage tube often dislodges from the fixed position within a few days after surgery due to the movement of the intestine (i.e., peristalsis), which occupies most of the space in the abdominal cavity. Because of this, the original purpose of the drainage tube, which is to drain unnecessary body fluids, blood or secretions, impossible so it virtually loses its function. The current method to solve this disadvantage is to secure the drainage tube to the peritoneum in the abdominal cavity using sutures, but the results of this method are not significantly different.

Recently, a method for each and effective fixation of the drainage tube has been attempted that the drainage tube is moved to the desired location and fixed through a route outside the abdominal cavity, that is, outside the peritoneum rather than inside the abdominal cavity.

The abdominal cavity is composed of the abdominal wall. The abdominal wall is largely composed of skin, subcutaneous fat layer, muscle layer surrounded by fascia in the front and back, fascia, preperitoneal fat layer and peritoneum. Since the drainage tube itself is made of a soft material, an assistive device is needed to move the drainage tube to the desired location through a path outside the peritoneum. Insertion of the assistive device by creating a tunnel parallel to the curvature of the abdominal cavity along the abdominal wall must be accomplished without damaging tissues and organs. Of the abdominal wall layers, the one that is least damaged and most easily moved is the preperitoneal fat tissue plane. The abdominal cavity is curved. If the assistive device is straight, damage to blood vessels and structures might occur while penetrating through the curved tissue.

The preperitoneal fat tissue plane is a physically weak tissue layer and can be easily peeled off. Accordingly, among the possible extraperitoneal routes, a route passing through the preperitoneal fat tissue plane is the easiest and safest way to reach the target location.

It is impossible with current technology to insert a drain tube through the peritoneum like this. So, the need for a device that can guide or assist for this purpose has been raised. However, there is no device among existing drainage tubes and related devices that can satisfy this need.

### [DETAILED DESCRIPTION OF THE INVENTION]

### [TECHNICAL PROBLEM]

Accordingly, one object of the present disclosure is to solve the above-noted disadvantages of the prior art, and to provide a drainage tube guide device that may guide a drainage tube to a target location while peeling the extraperitoneal fat layer without damaging tissues and organs.

### [TECHNICAL SOLUTION]

To solve the objects of the present disclosure, a drainage tube guide device is provided. The drainage tube guide device may include an outer inserter inserted along the abdominal wall; and an inner inserter inserted in a hollow of the outer inserter while peeling tissues in front of the outer inserter, wherein the outer inserter may include a head part extending along one direction; and a guide tube extending downward from the head part.

The head part may include an inlet hole formed on an upper surface, in communication with the hollow of the guide tube, so that the inner inserter or drainage tube can be inserted into the guide tube.

The inner inserter may include a coupling part coupled to the head pat; and a through-part inserted into the hollow of the inner inserter.

A protrusion protruding forward through the guide tube may be formed in the through-part.

The protrusion may be inclined downward toward an end.

The head part may include a coupling groove formed in each of both sides of the inlet hole formed in the head part, and the coupling part may include a hook portion protruding along both directions and fitted to the coupling groove.

The length of the guide tube may be 21cm to 31cm.

The guide tube may have a curved shape.

### [ADVANTAGEOUS EFFECTS]

The drainage tube guide device according to the present disclosure may guide the drainage tube by reaching the target location while peeling off the extraperitoneal fat layer without damaging tissues and organs.

Furthermore, the insertion and fixing of the drainage tube through the extraperitoneal route may be possible. Accordingly, drainage treatment may be possible for the desired period of time without displacement of the drainage tube due to intestinal movement or physical movement after surgery.

### [Description of Drawings]

FIG. 1 is a perspective view of a drainage tube guide device according to one embodiment;
FIGS. 2 and 3 are views to describe the coupling structure of an outer inserter and an inner inserter that are provided in a drainage guide device;
FIG. 4 is a view to describe a guide tube of an outer inserter according to one embodiment; and
FIG. 5 is a view to describe a protrusion of an inner inserter according to one embodiment.

### [DESCRIPTION OF SPECIFIC EMBODIMENTS]

Description will now be given in detail according to exemplary embodiments disclosed herein, with reference to the accompanying drawings. For the sake of brief description with reference to the drawings, the same or equivalent components may be provided with the same reference numbers, and description thereof will not be repeated.

Throughout the disclosure, each component can be provided as a single one or a plurality of ones, unless explicitly stated to the contrary. Terms such as "include" or "has" are used herein and should be understood that they are intended to indicate an existence of several components, functions or steps, disclosed in the specification, and it is also understood that greater or fewer components, functions, or steps may likewise be utilized.

Throughout the disclosure, each component can be provided as a single one or a plurality of ones, unless explicitly stated to the contrary. Terms such as "include" or "has" are used herein and should be understood that they are intended to indicate an existence of several components, functions or steps, disclosed in the specification, and it is also understood that greater or fewer components, functions, or steps may likewise be utilized.

Terms of respective elements used in the following description are terms defined taking into consideration of the functions obtained in the present invention. Therefore, these terms do not limit technical elements in the present invention. Further, the defined terms of the respective elements will be called other terms in the art. The singular expressions include plural expressions unless the context clearly dictates otherwise.

For the sake of brief description with reference to the drawings, the same or equivalent components may be provided with the same reference numbers, and description thereof will not be repeated.

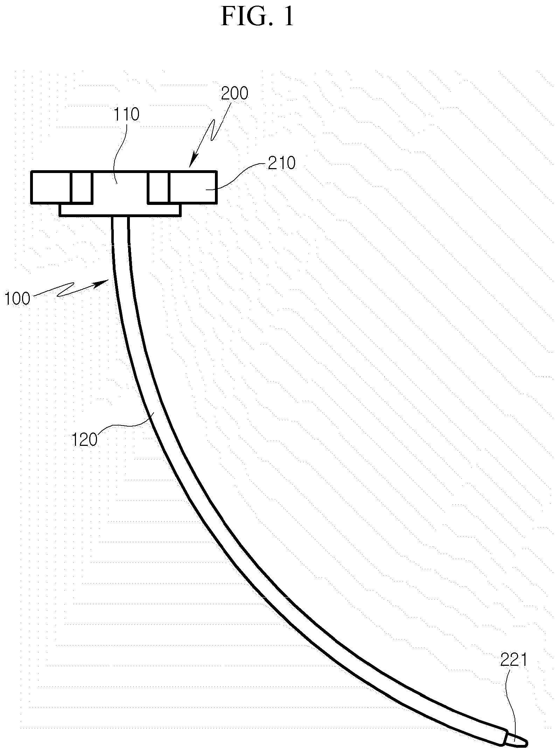

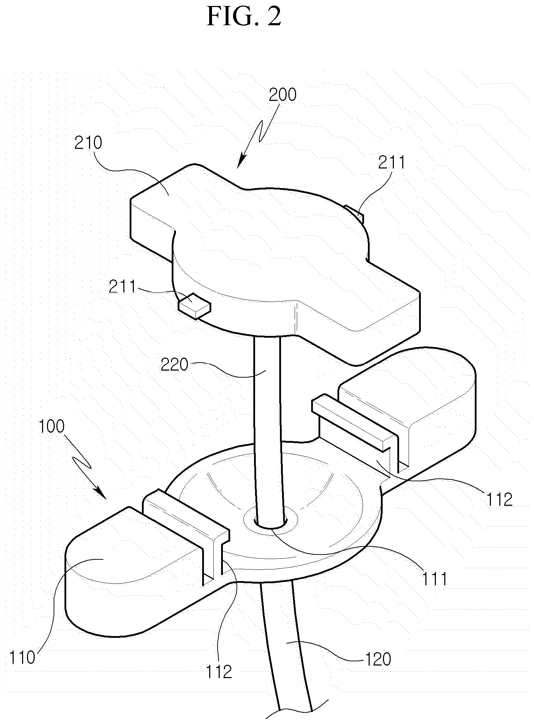

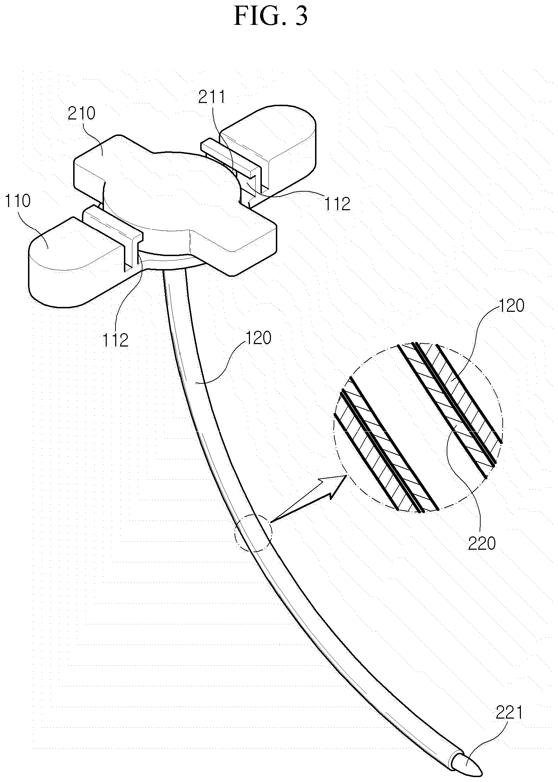

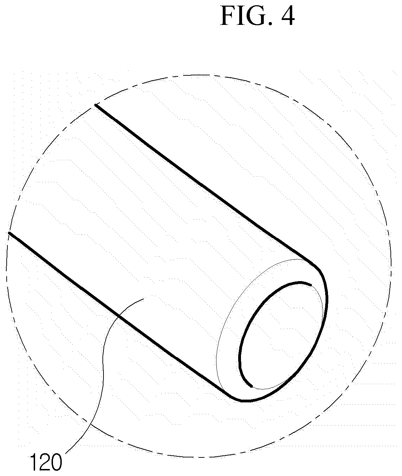

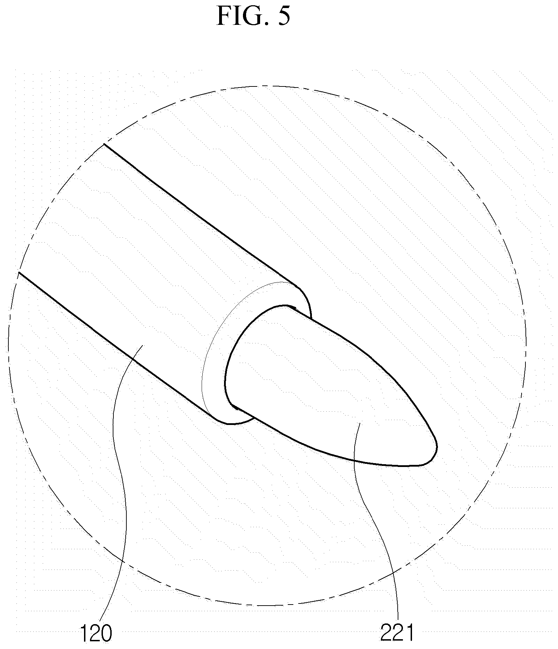

FIG. 1 is a perspective view of a drainage tube guide device according to one embodiment. FIGS. 2 and 3 are views to describe the coupling structure of an outer inserter and an inner inserter that are provided in a drainage guide device. FIG. 4 is a view to describe a guide tube of an outer inserter according to one embodiment. FIG. 5 is a view to describe a protrusion of an inner inserter according to one embodiment.

Referring to FIGS. 1 to 5, a drainage tube guide according to one embodiment may include an outer inserter 100 and an inner inserter 200.

The abdominal cavity is composed of the abdominal wall. The abdominal wall is largely composed of skin, subcutaneous fat layer, muscle layer surrounded by fascia in the front and back, fascia, preperitoneal fat layer and peritoneum.

As one example, the outer inserter 100 may be inserted parallel to the curve of the abdominal cavity along the abdominal wall.

As one example, the inner inserter 200 may be inserted into the hollow space of the outer inserter 100, while peeling tissues in front of the outer inserter 100. Once reaching a target location, the inner inserter 200 may be separated from the outer inserter 100 and the drainage tube may be inserted through an inlet hole 111 of the outer inserter 100.

As one example, the outer inserter 100 may include a head part 110 and a guide tube 120.

The head part 110 may be formed in a bar shape extending along both opposite directions as one example, and it may include an inlet hole 111 through which the inner inserter 200 or the drainage tube is introduced on the upper surface.

The inlet hole 111 may be in communication with a hollow space of the guide tube 120 as one example.

The head part 110 may be formed in various shapes, as one embodiment. As shown in the drawings, the various shapes may include a bar shape extending in both lateral directions, a spherical shape with a curved surface and a polyhedron shape with six or more sides.

As one example, the head part 110 formed in a shape of a rod or bar extending in both lateral directions may further include a plurality of grip grooves (not shown) that are directed inward, spaced apart from each other for the user's grip, in a shape of a rod or bar extending in both lateral directions, so that it can function as a handle.

The grip grooves (not shown) may be formed as a plurality of grooves recessed inward to fit the user's fingers for easy and comfortable grip when the user holds the head part 110.

The head part 110 may have coupling grooves 112 formed in both sides of the inlet hole 111 as one example.

As one example, the guide tube 120 may be formed in a hollow shape that is in communication with the inlet hole 111 and configured to guide the inner inserter 200 or drainage tube introduced through the inlet hole 111.

As one example, the guide tube 120 may extend downward from a lower surface of the head part 110 and extend in a spiral direction to form a curved surface as shown in the drawing.

The longitudinal axis of the abdominal cavity is usually about 30 to 50cm, the transverse axis is about 20 to 30 cm and the cross-sectional radius of the abdominal cavity is about 10 to 20cm, which vary depending on body type.

As one example, the guide tube 120 may have a curved shape with a radius of curvature of 12 to 42cm so as to move smoothly along the abdominal cavity, considering the curved shape of the abdominal cavity and the width and depth of the abdominal cavity.

The guide tube 120 may have the length of 21 to 31cm as one example, considering the width and depth of the abdominal cavity.

The guide tube 120 may be made of a plastic or metallic material having hardness that does not bend, as one example.

The guide tube 120 may be made of a soft plastic material that is bendable, as one example.

The inner inserter 200 may include a coupling part 210 and a through-part 220 as one example.

As one example, the coupling part 210 may be coupled to the head part 110 of the outer inserter 100.

The coupling part 210 may include a hook portion 211 protruding along both sides to be hooked to the coupling grooves 112 of the head part 110 as one example.

The coupling part 210 may be formed in a rod shape extending in both lateral directions as one example.

As one example, when the coupling part 210 is coupled to the head part 110, the both rod-shaped portions of the head part 110 and the rod-shaped portions of the coupling part 210 may be perpendicular coupled to each other. As one example, when the coupling part 210 is coupled to the head part 110, the rod-shaped portions of the head part 110 and the rod-shaped portions of the coupling part 210 may have a cross shape. Through this, the user's grip may be facilitated.

The through-part 220 may be inserted in the hollow space of the inner inserter 200 through the inlet hole 111 as one example.

As one example, one side of the through-part 220 may be coupled to the coupling part 210 and the other side thereof may have a protrusion 221 protruding forward through the guide tube 120.

The protrusion 221 may be formed downward to the end. That is, the protrusion 221 may have a round shape without sharp ends.

As one example, the protrusion 221 may be formed in a wedge shape without a sharp end.

Through the protrusion 221 without the sharp end, the target location may be reached without damaging tissues and organs, while the extraperitoneal fat layer is being peeled off.

While the inner inserter 200 is coupled to the outer inserter 100, the drainage tube may be smoothly introduced into the extraperitoneal route (e.g., extraperitoneal fat layer) through the protrusion 221 and damage to organs and blood vessels outside the peritoneum may be prevented.

The guide tube 120 of the present disclosure may spirally extend to have a length of 21 to 31cm and a curve radius of 12 to 42cm. This numerical limitation is a range that can vary based on body shape differences in order to reach the farthest target location from the abdominal drainage insertion site where the drainage tube is commonly inserted. insertion of an extraperitoneal drainage tube may minimize movement of the drainage tube due to intestinal peristalsis, thereby effectively draining the abdominal fluid for a long period of time. This allows for a high therapeutic effect than the conventional method of inserting and fixing the intraperitoneal drainage tube through the abdominal cavity.

The present disclosure relates to a drainage tube guide device including a protrusion 221 without a sharp end. According to the present disclosure, the target location may be reached while the extraperitoneal fat layer is being peeled without damage to tissues and organs. Therefore, the drainage tube is inserted through the retroperitoneum to drain the fluid inside the abdominal cavity, thereby making easier the process of inserting and fixing the drainage tube into the abdominal cavity through the existing surgical site and then improving the treatment effect.

Although the present invention has been described with reference to the exemplified drawings, it is to be understood that the present invention is not limited to the embodiments and drawings disclosed in this specification, and those skilled in the art will appreciate that various modifications are possible without departing from the scope and spirit of the present invention.

Further, although the operating effects according to the configuration of the present invention are not explicitly described while describing an embodiment of the present invention, it should be appreciated that predictable effects are also to be recognized by the configuration.

## Claims

1. A drainage tube guide device comprising:
an outer inserter inserted along the abdominal wall; and
an inner inserter inserted in a hollow of the outer inserter while peeling tissues in front of the outer inserter,
wherein the outer inserter comprises,
a head part extending along one direction; and
a guide tube extending downward from the head part.

2. The drainage tube guide device of claim 1, wherein the head part comprises an inlet hole formed on an upper surface, in communication with the hollow of the guide tube, so that the inner inserter or drainage tube can be inserted into the guide tube.

3. The drainage tube guide device of claim 2, wherein the inner inserter comprises,
a coupling part coupled to the head pat; and
a through-part inserted into the hollow of the inner inserter.

4. The drainage tube guide device of claim 3, wherein a protrusion protruding forward through the guide tube is formed in the through-part.

5. The drainage tube guide device of claim 2, wherein the protrusion is inclined downward toward an end.

6. The drainage tube guide device of claim 3, wherein the head part comprises a coupling groove formed in each of both sides of the inlet hole formed in the head part, and
the coupling part comprises a hook portion protruding along both directions and fitted to the coupling groove.

7. The drainage tube guide device of claim 1, wherein the length of the guide tube is 21cm to 31cm.

8. The drainage tube guide device of claim 1, wherein the guide tube has a curved shape.
